# EUROPEAN PATENT APPLICATION

(11) **EP 4 154 874 A1**
(43) Date of publication of application: **29.03.2023**
(21) Application number: 22197986.7
(22) Date of filing: 27.09.2022
(51) Int. Cl.: A61K 31/122, A61P 35/00

(54) **TETRATERPENES FOR USE IN CANCER THERAPY**

(30) Priority: 28.09.2021 US 202117487486
(71) Applicant: Shepherd, Samuel L., Leesville, SC 29070 (US)
(72) Inventor: Shepherd, Samuel L., Leesville, SC 29070 (US)
(74) Representative: Mewburn Ellis LLP

(57) **Abstract**

A method of treating a mammal afflicted with cancer includes preparing a therapeutic amount of tetraterpenes and administering the therapeutic amount of tetraterpenes to the mammal in need of such treatment. The tetraterpenes are selected from astaxanthin and direct derivatives thereof. The direct derivatives include a glycosidic or glucosidic form of astaxanthin containing at least one conjugated double bond and reactive keto-hydroxyl end groups. The therapeutic amount can be administered orally or topically to the mammal.

## Description

### BACKGROUND OF THE INVENTION

### Field of the Invention

The present invention relates to cancer treatment and therapy. More particularly, the present invention relates to the use of tetraterpenes for the treatment of such cancer or for cancer therapy. Furthermore, the present invention relates to a method of treating cancer through the administration of tetraterpenes, such as astaxanthin or derivatives of astaxanthin.

### Description of Related Art

Treatment of human cancer is an area of clinical medicine which presents many complications. These often present an array of sub optimal treatment choices. For example, prostate cancer, which is the most common cancer in men and the second leading cause of cancer death in men, can be treated surgically or medically, or with a combination of both, depending on the state of advance of the cancer. Other than hormonal therapy, no agents are available to treat prostate cancer that are without substantial toxicity. Frequently, cancer occurs in debilitated patients who are either not surgical candidates are who would not tolerate conventionally available chemotherapeutic agents.

Radiation therapy (e.g. X-ray treatment) is one modality available to treat selected cancers. It is used at many stages in the treatment of prostate cancer. For example, it can be used as an alternative to surgery to treat localized prostate cancer. X-ray therapy can also be used in metastatic prostate cancer to treat local deposits of tumors that threaten key organs, such as the spinal cord. Radiation can be useful to treat a number of cancers in settings, such as to attempt to cure the local tumor, along with the symptomatic management of troublesome metastatic disease.

Osteosarcoma is an example of a cancer primarily to the bone. This can metastasize and, as such, remains a major tumor problem in children. A subtype of osteosarcoma, chondrosarcoma, does not metastasize. However, chondrosarcoma often recurs locally after surgical excision. As such, it can require more than surgery alone. Glioblastomas are malignant tumors which do not metastasize generally, but which are commonly beyond surgical cure. These have median patient survivals of less than one year. A rapidly growing primary tumor that is not surgically curable can cause significant morbidity and mortality without necessarily metastasizing. In such a case, effective chemotherapy is needed.

With the discovery of PD-L1 by Dr. James Allison of M.D. Anderson Hospital and characterized at the Mayo Clinic as an immune regulatory molecule, much research has been undertaken. PD-L1 was identified as a ligand of PDL. Several human cancer cells expressed high levels of PD-L1. It has been shown that blocking of PD-L1 reduced the growth of tumors in the presence of immune cells. As such, it was concluded that PD-L1 terminates with the C terminus of the tyrosine amino acid. PD-L1 helps tumor cells evade anti-tumor attack by the immune system T-cells.

PD-L1 binds to its receptor PD-1 that is found on activated T-cells, B cells, and myeloid cells. This inhibits the T-cell attack of the tumor cell. It is been found that the carboxyl group on the end of the PD-L1 is reactive via the A2 pathways with tetraterpenes. First, the keto-hydroxyl functionality of the tetraterpene reacts directly to the carboxyl group of the PD-L1 and/or secondly, the conjugated endine structure of the tetraterpene reacts directly with the carboxyl group of the PD-L1. These reactions cause complete inactivation of the PD-L1 and further inhibits the reaction of PD-L1. This allows for T-cell identification and destruction of the cancer cell.

Astaxanthin belongs to a larger class of chemical compounds known as tetraterpenes (as a tetraterpenoid) built from five carbon precursors, isopentny diphosphate and dimethylallyl diphosphate.

Astaxanthin is classified as a xanthophyll. This term was originally direct derived from a word meaning "yellow leaves" since yellow plant leaf pigments were the first recognized of the xanthophyll family of carotenoids. Astaxanthin is currently employed to to describe carotenoids compounds that have oxygen-containing components, hydroxyl (-OH) or ketone (C=O), such as the zeaxanthin and canthaxanthin. Indeed, astaxanthin is a metabolite of zaxanthin and/or canthaxanthin, both containing hydroxyl and ketone functional groups.

Like many carotenoids, astaxanthin is a lipid-soluble pigment. Its red-orange color is due to the extended chain of conjugated (alternating double and single) double bonds at the center of the compound. This chain of conjugated double bonds is also responsible for the antioxidant (electron donation) function of astaxanthin (as well as other carotenoids) since it results in a region of decentralized electrons that can be donated to reduce a reactive oxidizing molecule.

More generally, a chemotherapeutic agent which possesses little or no toxicity, which is inexpensive to obtain or manufacture, which is well tolerated by the patient, and which is easily administered would be a desirable addition to the array of therapeutic modalities available to the oncologist. Such a chemotherapeutic agent would find application and treatment of cancers which are metastatic, locally expanding or locally invasive. Additionally, such an agent could be useful if it were to sensitize the cancer to radiation or chemotherapy. Thus, the cancer could respond more readily to X-ray and chemotreatment.

Astaxanthin, as a free radical scavenger/antioxidant, is 400 times more reactive as an antioxidant than betacarotene. Astaxanthin is not only the world's strongest natural antioxidant, astaxanthin is also a safe and natural anti-inflammatory. Astaxanthin is incredibly potent and well-rounded in its antioxidant activity. As an example, in an antioxidant test identified as "singlet oxygen quenching", astaxanthin has been shown to be 550 times stronger than vitamin E, 800 times stronger than CoQ10 and 6000 times stronger than vitamin C.

In the past, various patents and patent publications have been directed to the production of astaxanthin. For example, U.S. Patent Publication No. 2010/0285557, published on November 11, 2010 to Martin et al., pertains to a method for the efficient production of carotenoids. In particular, this invention is directed to a method for producing carotenoid and carotenoid-containing cells, especially astaxanthin and astaxanthin-containing cells, by generating mutant microorganisms belonging to the photoautotrophic algae of the class chlorophyceae and culturing the same.

U.S. Patent No. 7,063,957, issued on June 20, 2006 to F. Chen, discloses a method for producing ketocarotenoid astaxanthin by the green microalga Chlorella zofingiensis in dark-heterotrophic cultures so as to provide excellent growth and high-yield astaxanthin production on glucose-supplemented media in the dark.

U.S. Patent Publication No. 2005/0124032, published on June 9, 2005 to De La Fuente Moreno et al., teaches a method of producing of astaxanthin by fermenting selected strains of Xanthophyllomyces dendrorhous. These strains are selected based on: (i) resistance to inhibitors of steroid synthesis, to inhibitors of respiration and to compounds that induce the formation of free radicals; (ii) color intensity of the colony and production of carotenoids on solid medium; (iii) production of astaxanthin in darkness; (iv) production of astaxanthin in conditions with a raised temperature; and (v) production of astaxanthin with carbon sources other than glucose.

U.S. Patent Publication No. 2004/0077036, published on April 22, 2004 to Thomas et al., provides a process to produce astaxanthin from haematococcus biomass. In particular, a modified nutrient medium containing four nitrogen sources is used for culturing the algae. Green motile cells produced are converted into dormant red cysts which are chilled and stressed for vigorous multiplication. Nutrients are added gradually and the initial germination is carried out without carbon dioxide sparging. The dilution stage is also effected in the absence of carbon dioxide. The stressed red cysts are regerminated and the cycle repeated to produce a biomass enriched with astaxanthin.

U.S. Patent No. 6,022,701, issued on February 8, 2000 to Boussiba et al., discloses a procedure for large-scale production of astaxanthin from haematococcus. In this process the haematococcus cells are cultivated under conditions suitable for optimal vegetative growth of such cells and the cells are collected and cultivated under conditions suitable for optimal induction and accumulation of astaxanthin in the cells. The cells are inoculating into a growth solution containing essentially a carbon source and growing the cells at a temperature of below 35 C.

U.S. Patent No. 11,065,269, issued on July 20, 2021 to S. L. Shepherd, describes a method of using astaxanthin for the treatment of diseases. In particular, this patent describes a method of treating the subject afflicted with cancer, arthritis, diabetes, lupus, fibromyalgia or dementia. The method includes administering a therapeutic amount of a glycosidic astaxanthin to the subject in need of such treatment. The glycosidic astaxanthin is prepared by reacting astaxanthin with a monosaccharide at a temperature so as to produce the glycosidic astaxanthin. The therapeutic amount is not greater than 0.1 mg per kilogram of body weight per day. The glycosidic astaxanthin can be added to a carrier material, such as in edible material, a pill, or an oil.

U.S. Patent No. 9,572,783, issued on February 21, 2017 to C. W. Lou, shows the use of xanthophylls for the treatment of cancers. In particular astaxanthin and/or an ester thereof is used to inhibit tumor cell growth. In particular, this provides a method for preventing or treating cancers includes the step of administering to a subject in need thereof a composition comprising a therapeutic effect of amount of at least one type of xanthophyll.

U.S. Patent No. 6,773,708, issued on August 10, 2004 to Lignell, describes the use of xanthophylls for the treatment of autoimmune diseases, along with chronic viral and intracellular bacterial infections. This is a medicament for suppression of excessive Th1 cell mediated immune responses and stimulation of Th2 cell mediated immune responses in a patient during ongoing infection and/or inflammation. The preferred type of xanthophyll is astaxanthin, particularly in a form esterified with fatty acids obtained by culturing the algae hamematococcus.

It is an object of the present invention provide a method for treating cancer that uses a chemotherapeutic agent which possesses little or no toxicity.

It is another object of the present invention to provide a method for treating cancer with a chemotherapeutic agent that is inexpensive to obtain or manufacture.

It is another object of the present invention provide a method of treating cancer which utilizes a chemotherapeutic agent that is well-tolerated by the patient.

It is a further object of the present invention to provide a method of treating cancer which can be easily administered.

It is a further object of the present invention to provide a method of treating cancers which can be used with cancers that are metastatic, locally expanding or locally invasive.

It is still further object of the present invention to provide a method of treating cancers which can sensitize the cancer to radiation or chemotherapy.

It is still a further object of the present invention provide a method of treating cancer which causes the cancer to respond more readily to X-ray and chemotreatment.

These and other objects and advantages of the present invention will become apparent from a reading of the attached specification and appended claims.

### BRIEF SUMMARY OF THE INVENTION

The present invention is a method of treating a mammal afflicted with cancer. This method includes the steps of: (1) preparing a therapeutic amount of tetraterpenes; and (2) administering the therapeutic amount of tetraterpenes to the mammal in need of such treatment. The tetraterpenes are selected from the group consisting of astaxanthin and direct derivatives thereof. The direct derivatives include a glycol acidic, glycosidic or glucosidic form of astaxanthin containing at least one conjugated double bond and reactive keto-hydroxyl and groups.

The cancer has staged term of I to IV cancer. The step of administering can be either orally or topically administrating the therapeutic amount to the mammal. The preferred mammal would be a human. The therapeutic amount is between 0.1 mg per kilogram of body weight per day to 100 mg per kilogram of body weight per day. In the preferred embodiment, the therapeutic amount is between 0.5 milligrams per kilogram of body weight per day to 2 mg per kilogram of body weight per day. The tetraterpenes are selected from the group consisting of astaxanthin, glycosidic astaxanthin, liposomal astaxanthin and mixtures thereof. The cancer cells are selected from the group including non-small cell lung cancer, melanoma, Hodgkin's lymphoma, bladder cancer, kidney cancer, breast cancer, prostate cancer, colon cancer, glioblastoma, astrocytoma, sarcoma and combinations thereof.

The present invention also includes a method of sensitizing cancer to radiation and a method of inhibiting the growth of cancer cells.

The present invention provides methods of treating certain cancers that includes the steps of administering an effective amount of selected tetraterpenes to a mammal having a cancer susceptible to such treatment. Most preferably, the cancer is a PD-L1 expressed cancer such as ademocarcinoma of the prostate or colon-rectal cancers, lung cancer, melanoma, basal cell, glioblastoma, or a sarcoma such as osteosarcoma. Preferably, the administration is accomplished by the oral route and is given one to three times per day. The tetraterpenes or adjacent are direct derivatives of such. These derivatives include all glycosidic, liposomal or glucosidic forms. Preferably, the mammal treated is a human, although in the method of the present invention can have other applications, such as veterinary use.

This foregoing Section is intended to describe, with particularity, the preferred embodiments of the present invention. It is understood that modifications to these preferred embodiments can be made within the scope of the present claims. As such, this Section should not to be construed, in any way, as limiting of the broad scope of the present invention. The present invention should only be limited by the following claims and their legal equivalents.

### DETAILED DESCRIPTION OF THE INVENTION

The present invention includes methods of treating selected cancers. This method comprises administering an effective or therapeutic amount of a tetraterpenes to a mammal having the cancer. The cancer is selected from the group including PD-L1 cancer or cancers exhibiting local pH of less than 7.35. The tetraterpenes is selected from the group consisting of astaxanthin, glycosidic astaxanthin, glucosidic astaxanthin or liposomal astaxanthin.

Astaxanthin is a keto-carotenoid. It belongs to a larger class of chemical compounds known as terpenes (as a tetraterpenoid) built from five carbon precursors, isopentnyl diphosphate and dimethylallyl diphosphate. Astaxanthin is classified as a xanthophyll, but currently employed to describe carotenoid compounds that have oxygen -containing components, hydroxyl (-OH) or ketone (C=O), such as xeaxanthin and canthaxanthin. Astaxanthin is a metabolite of xeaxanthin and/or canthaxanthin containing both hydroxyl and ketone functional groups.

Like many carotenoids, astaxanthin is a lipid-soluble pigment. The red-orange color of astaxanthin is due to the extended chain of conjugated (alternating double and single) double bonds at the center of the compound. This chain of conjugated double bonds is also responsible for the antioxidant function of astaxanthin (as well as other carotenoids) since it results in a region of decentralized electrons that can be donated to reduce a reactive oxidizing molecule.

Tetraterpenes are organic compounds constructed of multiples of the 5-carbon hydrocarbon isoprene unit. Additionally, higher order tetraterpenes exist and there is no set upper limit on how many isoprene units a tetraterpenes may include. For example, natural rubber is a tetraterpene with approximately sixty isoprene units.

Generally, prostate cancers and colon cancers are adenocarcinomas having PD-L1 expression. However, in roughly 5% of the cases, either of these cancers are some other type. An example of an astrocytoma is a glioblastoma. Examples of sarcomas include osteosarcomas, fibrosarcomas, and rhabdomyosarcomas. The cancer to be treated can be metastatic, locally expanding or locally invasive.

The administration of the tetraterpenes can be accomplished by any number of routes, but usually either oral or topical administration is used. The oral route is preferable. The term "oral route" means that the patient would swallow the drug. The term also includes gavage, which is a preferred delivery in nonhuman animals.

Preferably, the mammal to be treated as a human. For humans, the effective amount of tetraterpenes is selected from a range from about 0.1 mg per kilogram of body weight of the mammal per day to about 100 mg per kilogram of body weight of the mammal per day. The preferred effective amount is in the range of about 0.5 mg per kilogram of body weight of the mammal to about 2.0 mg per kilogram of body weight of the mammal per day. A single daily administration is typical. However, the dose can be given in two, three or more times a day. The tetraterpenes are usually administered on a long-term basis or chronically for weeks, months or years.

The tetraterpenes of the present invention can be stored at room temperature, preferably protected from bright light and away from direct heat. The pleasant taste and odor of these tetraterpenes permit their use as diluted, as mixed with liquid, or is given with food. Juices, dairy products, and mashed fruits make particularly suitable vehicles for oral consumption. Alternatively, the tetraterpenes can be presented in encapsulated form, in a syrup, a gel, a lozenge, or other conventional means for delivering oral medication. Additionally, the tetraterpenes can be diluted to a preselected concentration in a liquid. The patient is given (or instructed to take) a specified volume, such as a tablespoonful, which can be taken alone or mixed in liquid or semi-liquid consumable or in capsule form.

The present invention also includes a method of sensitizing a cancer to chemotherapy and/or radiation. This method includes the step of administering an effective or therapeutic amount of a chosen tetraterpene to a mammal having the cancer. The cancer is preferably either PD-L1 expressed to prosthetic or colonic.

The present invention also includes a method of inhibiting the growth of cancer cells. This method includes applying an effective amount of a selected tetraterpenes to the cancer cells. The tetraterpenes have the ability to donate electrons to the hydroxyl-free radical having a net (zero) charge (conjugated acid) so as to make it acidic in activity. The donated electron to the free radical imparts a negative charge, making it a hydroxyl ion. This action raises the pH to over 8.0 thereby causing tremendous stress within the cancer cell and serves to initiate apoptosis.

The application of the tetraterpenes to the cell is either direct or indirect. The cells can be located in a mammal. When the cells are located in a mammal, the application is usually indirect or preferably oral. Direct application can be made topically. The mammal is preferably a human. The application is preferably wants to three times per day.

As used herein, the term "therapeutic amount" means a dosage sufficient to produce a desired result. The desired result can be a subjective or object of improvement in the recipient of the dosage, a decrease in tumor size, a decrease in the rate of growth of cancer cells, and/or an enhanced sensitivity of cancer cells to radiation. The term "treating cancer", "therapy" and the like mean generally any improvement in the mammal having the cancer wherein the improvement can be ascribed to treatment with the tetraterpenes. The improvement can be either subjective or objective. For example, if the mammal is human, the patient may note improved vigor or vitality or decreased pain as subjective symptoms of improvement or response to therapy. Alternatively, the clinician may notice a decrease in tumor size or tumor burden based on a physical exam, based upon laboratory parameters, on tumor markers, on radiographic findings.

Some laboratory signs that the clinician may observe for response to therapy include normalization of tests such as C Reactive Protein, white blood cell count, red blood cell count, platelet count, erythrocyte sedimentation rate, and various enzyme levels, such as transaminases and hydrogenase. Additionally, the clinician may observe a decrease in a detectable tumor marker, such as a prosthetic specific antigen (PSA), C Reactive Protein (CRP), or carcino embryonic antigen (CEA). Alternatively, other tests can be used to evaluate object of improvement, such as sonograms, nuclear magnetic resonance testing, and positron and emissions testing.

The term "inhibiting the growth of cancer cells" can be evaluated by any accepted method of measuring whether the growth of the cancer cells has been slowed or diminished. This includes direct observation and indirect evaluation, such as subjective symptoms or objective signs. The term
"sensitizing a cancer to chemotherapy or radiation" means that, as a result of treatment with the tetraterpenes, the cancer is more likely to respond to chemo or radiation exposure then it would have been without treatment with the tetraterpenes. The term "respond to radiation" means that the cancer is expected to either regress or slow its growth subsequent to radiation therapy.

In living animals, metabolic rate decreases as body size increases. For drugs that undergo metabolism or act by altering a metabolic event, drug dosing must take this into consideration. Body surface area correlates better with metabolic rate than does body weight. For this reason, it is best to use body surface area to convert dosing in one species to that of another. There are correlation factors that exist that use body surface area to convert mouse doses to that of other species. A rat would get about one-half of the mouse dose. A monkey, which weighs about three kilograms, would get about one-fourth of the mouse dose. A dog which weighs about eight kilograms would get one-sixth of the mouse dose. A human would get approximately one-twelve of the mouse dose. Thus, a fourteen gram dose in a mouse correlates to slightly over one gram in a human. The 126 gram dose in a mouse correlates to about eleven grams in a human.

The present invention provides a method of treating cancer to the administration of tetraterpenes, namely, astaxanthin or derivatives of astaxanthin, including, but not limited to, glycosidic astaxanthin. The cancers to be affected by this method include PD-L1 expressed cancers and cancers having high concentrations of Reactive Oxygen Species (ROS). PD-L1 is a protein that is expressed by certain cancers. This expression helps keep immune cells, such as T and B cells from attacking non-harmful cells in the body. Normally, the immune system fights foreign substances, such as viruses and bacteria, that do not express PD-L1 and not healthy cells. Some cancer cells have high amounts of PD-L1. This allows the cancer cells to "trick" the immune system and thus avoid being attacked as foreign, harmful substances. The cancers affected by PD-L1 expression include, but are not limited to, non-small cell lung cancer, melanoma, Hodgkin's lymphoma, bladder cancer, kidney cancer, breast cancer, prostate cancer, colon cancer, glioblastoma, astrocytoma, or sarcoma. These cancers also contain high concentrations of Reactive Oxygen Species, namely, hydroxyl and peroxyl free radicals. These free radicals lower the pH of the environment and thereby promote the proliferation of the environment and thereby promote the proliferation of growing cancer cells.

The present invention also provides a method of sensitizing or weakening the cancer prior to or in conjunction with radiation or chemo treatments. This method includes administering an effective amount of a tetraterpenes to a mammal having the cancer wherein the tetraterpene is selected from the above group. The present invention further provides a method of inhibiting the growth of cancer cells through the conversion of the hydroxyl free radical, which is a conjugated acid, to the hydroxyl ion, a strong base. This action results in the cellular pH to be increased from less than 7.35 to greater than 8.8 at the cellular level. This increase in pH results in apoptosis of the cancer cell.

The adsorption of the tetraterpene is enhanced by the glycosidic or liposomal structure of the astaxanthin. When the tetraterpenes are used to sensitize cancer, the tetraterpenes will contain at least one site of unsaturation. The tetraterpenes are drawn through the glucose channels of the cancer cells. The tetraterpenes donate an electron to the hydroxyl free radical so as to convert the free radical into the hydroxyl ion and thereby raise the cellular pH of the cancer cell in order to cause rapid apoptosis. The hydroxyl and/or keto species on the ends of the tetraterpenes react with the PD-L1 C terminus. The PD-L1 C terminus is a reactive carboxyl group. The tetraterpenes chemically attaches to the PD-L1 thereby inhibiting the PD-L1 for making contact with the PD1 of the T-cell. This allows for T-cell identification and destruction of the cancer cell.

The foregoing disclosure and description of the invention is illustrative and explanatory thereof. Various changes in the details of the described method can be made within the scope of the present invention without departing from the true spirit of the invention. The present invention should only be limited by the following claims and their legal equivalents.

## Claims

1. A composition for use in a method of treating a mammal afflicted with cancer, the composition comprising a therapeutic amount of tetraterpenes, the tetraterpenes being selected from the group consisting of astaxanthin and direct derivatives thereof, the direct derivatives including a glycosidic or glucosidic form of astaxanthin containing at least one conjugated double bond and reactive keto-hydroxylend groups.

2. The composition for use of claim 1, wherein the cancer is staged from I to IV cancer.

3. The composition for use of claim 1 or 2, wherein the composition is administered orally or topically.

4. The composition for use of any one of claims 1 to 3, wherein the mammal is a human, and/or the therapeutic amount is between 0.1 mg per kilogram of body weight per day to 100 mg per kilogram of body weight per day, preferably between 0.5 mg per kilogram of body weight per day to 2 milligrams per kilogram of body weight per day.

5. The composition for use of any one of claims 1 to 4, wherein the tetraterpenes are selected from the group consisting of astaxanthin, glycosidic astaxanthin, liposomal astaxanthin and mixtures thereof.

6. A composition for use in a method of sensitizing cancer to radiation, the composition comprising a therapeutic amount of tetraterpenes, the tetraterpenes having at least one site of unsaturation.

7. The composition for use of claim 6, wherein the cancer is staged from I to IV cancer.

8. The composition for use of claim 6 or 7, wherein the mammal is a human.

9. The composition for use of any one of claims 6 to 8, wherein the tetraterpenes are selected from the group consisting of astaxanthin, glycosidic astaxanthin, liposomal astaxanthin and mixtures thereof.

10. A composition for use in a method of inhibiting a growth of cancer cells, the comprising a therapeutic amount of tetraterpenes, the tetraterpenes least having at least one site of unsaturation.

11. The composition for use of claim 10, the composition is administered orally.

12. The composition for use of claim 10 or 11, wherein the mammal is a human.

13. the composition for use of any one of claims 10 to 12, wherein the therapeutic amount is between 0.1 mg per kilogram per day to 100 mg per kilogram per day, preferably between 0.5 mg per kilogram of body weight per day to 2 milligrams per kilogram of body weight per day, wherein optionally the therapeutic amount is applied once per day.

14. The composition for use of any one of claims 10 to 13, wherein the tetraterpenes are selected from the group consisting of astaxanthin, glycosidic astaxanthin, liposomal astaxanthin and mixtures thereof.

15. The composition for use of any one of claims 10 to 14, wherein the cancer cells are selected from the group consisting of non-small cell lung cancer, melanoma, Hodgkin's lymphoma, bladder cancer, kidney cancer, breast cancer, prostate cancer, colon cancer, glioblastoma, astrocytoma, sarcoma and combinations thereof.
